**Europäisches Patentamt**

(19) **European Patent Office**

**Office européen des brevets**

(11) Veröffentlichungsnummer: **0 019 808**
**B1**

## (12) EUROPÄISCHE PATENTSCHRIFT

(45) Veröffentlichungstag der Patentschrift:
**29.06.83**

(51) Int. Cl.³: **A 61 K 35/78**

(21) Anmeldenummer: **80102707.9**

(22) Anmeldetag: **16.05.80**

(54) **Arzneimittel zur Krebsbekämpfung.**

(30) Priorität: **22.05.79 DE 2920631**

(43) Veröffentlichungstag der Anmeldung:
**10.12.80 Patentblatt 80/25**

(45) Bekanntmachung des Hinweises auf die Patenterteilung:
**29.06.83 Patentblatt 83/26**

(84) Benannte Vertragsstaaten:
**AT BE CH FR GB IT LI LU NL SE**

(56) Entgegenhaltungen:
**DE-C-434 633**
**CHEMICAL ABSTRACTS, Band 53, Nr. 22, 25. November 1959, Zusammenfassung Nr. 22745h, Columbus, Ohio, US, E. VEVERA: «Contributions to the new edition of the «Homöopatisches Arzneibuch» second edition»**
**CHEMICAL ABSTRACTS, Band 50, Nr. 2, 25. Januar 1956, Zusammenfassung Nr. 1194h, Columbus, Ohio, US, R. KRAHL et al.: «An active principle from Drosera rotundifolia and its pharmacological investigation»**
**CHEMICAL ABSTRACTS, Band 48, Nr. 22, 25. November 1954, Zusammenfassung Nr. 13800a, Columbus, Ohio, US, F. K. FITZPATRICK: «Plant substrances active against Mycobacterium tuberculosis»**
**Brockhaus Enzyklopädie, 1966, «Tierfangende Pflanzen» und «Aronstabgewächse»**

(73) Patentinhaber: **Keller, Helmut, Dr.med., Blumenstrasse 32, D-8646 Nordhalben (DE)**

(72) Erfinder: **Keller, Helmut, Dr.med., Blumenstrasse 32, D-8646 Nordhalben (DE)**

(74) Vertreter: **Patentanwälte Czowalla . Matschkur + Partner, Königstrasse 1, D-8500 Nürnberg 106 (DE)**

Die Akte enthält technische Angaben, die nach dem Eingang der Anmeldung eingereicht wurden und die nicht in dieser Patentschrift enthalten sind.

Arzneimittel zur Krebsbekämpfung

Die Erfindung bezieht sich auf ein Arzneimittel zur Krebsbekämpfung, wobei Krebs eine sehr häufig auftretende, stets tödlich endende Krankheit ist, die durch ein entartetes Wachstum maligner Tumorzellen den Organismus mit unaufhaltsam wuchernden und ausstreuenden bösartigen Tumoren durchdringt und dadurch zur Zerstörung lebenswichtiger Organe oder Bestandteile des Organismus führt.

Es sind chemische Mittel, sogen. Zytostatika, bekannt, die das Zellwachstum hemmen und infolge des schnellen ungeordneten Wachstums maligner Tumoren an diesen stärker angreifen als am gesunden Gewebe. Doch führen diese Zytostatika nicht zu einer dauerhaften Heilung der Krebserkrankung, sondern im günstigsten Fall nur zu einer vorübergehenden Eindämmung und Verzögerung des Tumorwachstums, so dass ein erfolgreicher Einsatz von Zytostatika bei der Krebsbekämpfung nur zeitlich beschränkt möglich ist. Ausserdem schädigen diese Zytostatika infolge ihrer Wachstumshemmung auch die gesunden Zellen des Organismus erheblich, so dass ihre Anwendung mit schwersten schädigenden Nebenwirkungen verbunden ist. Überdies hat sich die Anwendung von Zytostatika nur bei bestimmten Tumorarten als erfolgversprechend erwiesen. Insgesamt sind also bisher keine chemischen Mittel für eine dauerhaft wirksame Krebsbekämpfung bekannt.

Im Rahmen der Erfindung wurde nun festgestellt, dass ein Arzneimittel zur Krebsbekämpfung, das den Saft fleischfressender Pflanzen, gegebenenfalls zusammen mit pharmazeutisch verträglichen Trägern und/oder Füllstoffen enthält, bösartige Tumoren vollständig auflöst.

Diese krebsbekämpfende Wirkung, die in umfänglichen Versuchen inzwischen eindeutig nachgewiesen werden konnte, ist ausserordentlich überraschend, obgleich einige fleischfressende Pflanzen, nämlich Drosera rotundifolia, Pinguicula vulgaris und Pinguicula alpina, d.h. drei spezielle Arten des Sonnentaus bereits seit Jahrhunderten als Arzneimittel zur Behandlung beispielsweise bei Arteriosklerose oder von Husten und Asthma, bekannt waren. Die dort wirksamen, in der pharmakologischen Literatur nach wie vor als unbekannt bezeichneten Wirkstoffe dürften jedoch nach den ersten vorliegenden Untersuchungen andere Bestandteile dieser Sonnentauarten sein als diejenigen, die gemäss der vorliegenden Erfindung eine ausserordentlich günstige selektive Bekämpfung von Krebs, einschliesslich der Krebsmetastasen bewirken. Nicht zuletzt ergibt sich dies daraus, dass nach den bisherigen Untersuchungen auch gerade andere fleischfressende Pflanzen als gerade Sonnentau besonders starke Wirksamkeit als Krebsbekämpfungsmittel zeigen.

Die krebsbekämpfende Wirkung, die nach gesicherten Untersuchungsergebnissen in den meisten Fällen dazu führt, dass bösartige Tumore vollständig aufgelöst werden, ohne dass auch nur die geringste Schädigung von gesundem Gewebe beobachtet werden konnte, ist bei rückblickender Betrachtung und Kenntnis der Wirkung möglicherweise aus der Tatsache verständlich, dass einerseits maligne, d.h. bösartige Tumore in ihrem Zellaufbau sehr der Primitivität niedriger Tiere ähnlich sind, und andererseits die fleischfressenden Pflanzen alle in der Lage sind, die zwischen ihren Blättern eingefangenen Insekten zu verdauen. Somit stellen die fleischfressenden Pflanzen proteolytische Fermente her, die in der Lage sind, primitive Epithelien anzugreifen. Im Rahmen der erfinderischen Bemühungen angestellte Versuche, von denen einer nachstehend beschrieben ist, haben nunmehr gezeigt, dass diese Fähigkeit zur Auflösung primitiver Epithelien auch zur Auflösung bösartiger Tumore im lebenden Organismus führt und eine vollständige Krebsheilung ermöglicht.

Neben dem oral einzunehmenden Saft der fleischfressenden Pflanzen, deren Wirksamkeit auch bereits eindeutig bei der Bekämpfung von Krebs bei Menschen nachgewiesen werden konnte, soll in Ausgestaltung der Erfindung auch eine Injektionslösung beispielsweise mit einer Natrium-Chlorit-Trägerlösung verwendet werden. Derartige Injektionslösungen aus Bestandteilen von fleischfressenden Pflanzen sind dabei ein Novum, nachdem ja bisher meist Extrakte aus den getrockneten Blättern der Pflanzen für die Behandlung der völlig anders gearteten eingangs beschriebenen Krankheiten eingesetzt worden sind.

In einem Versuch wurde zur Herstellung des Saftes der Carnivoren, d.h. fleischfressenden Pflanzen, speziell Dionaea muscipula, auch Venusfliegenfalle genannt, und Drosera rotundifolia, auch Sonnentau genannt, verwendet. Die Pflanzen wurden mit einem scharfen Messer zerkleinert und durch einen handelsüblichen Mikrofilter gepresst. Das so gewonnene Exprimat wurde zur Herstellung einer Injektionslösung mit 0,9%iger NaCl-Lösung verdünnt, wobei ein Verdünnungsverhältnis von 10 ml Exprimat mit 4 ml der 0,9%igen NaCl-Lösung angesetzt wurde.

Als Versuchstiere wurden Hamster, alle Kinder der gleichen Elterntiere, verwendet, die in zwei gleichgrosse Gruppen unterteilt wurden, deren eine Gruppe als Kontrolltiere diente.

Bei der anderen Gruppe wurden in Narkose die Backentaschen ausgestülpt und mittels einer kleinen Inzision eine subepitheliale Nische geschaffen, in die bei je 12 Hamsterbackentaschen Lymphosarkom- und Plattenepithelkarzinomgewebe von je etwa 3 mm Durchmesser implantiert wurde. Durch das einsetzende Tumorwachstum hatten alle Implantate nach 9 Wochen einen Durchmesser von 10–12 mm.

Nach dieser 9-wöchigen Wachstumszeit der Tumoren wurden den krebskranken Tieren in zweitägigem Abstand insgesamt 15 Injektionen von je 1,5 ml der oben beschriebenen Injektionslösung intraperitoneal verabreicht. Zum Vergleich wurde den je 6 anderen Kontrolltieren ebenso jeden zweiten Tag 1,5 ml reiner 0,9%iger NaCl-Lösung injiziert.

Nach den 15 Injektionen zeigte sich bei allen behandelten Tieren eine Verkäsung und völlige Auflösung der Tumoren. Die Tumoren hatten sich schliesslich vollständig zurückgebildet. Dagegen verstarben die nicht mit den erfindungsgemässen Arzneimittel behandelten Tiere alle, nachdem eine Aussaat der Tumoren über den ganzen Körper stattgefunden hatte.

**Patentansprüche**

1. Arzneimittel zur Krebsbekämpfung, dadurch gekennzeichnet, dass es den Saft fleischfressender Pflanzen mit Ausnahme von Drosera rotundifolia, Pinguicula vulgaris und Pinguicula alpina – ggfls. zusammen mit pharmazeutisch verträglichen Trägern und/oder Füllstoffen – enthält.

2. Arzneimittel nach Anspruch 1, dadurch gekennzeichnet, dass es den Saft der Blätter der fleischfressenden Pflanzen enthält.

3. Arzneimittel nach Anspruch 1 oder 2, dadurch gekennzeichnet, dass es den Saft von Dionaea muscipula, auch Venusfliegenfalle genannt, enthält.

4. Arzneimittel nach einem der Ansprüche 1 bis 3, dadurch gekennzeichnet, dass es die in den fleischfressenden Pflanzen enthaltenen proteolytischen Fermente enthält.

5. Verfahren zur Herstellung eines Arzneimittels nach einem der Ansprüche 1 und 4, dadurch gekennzeichnet, dass man die fleischfressenden Pflanzen, bzw. deren Blätter, zerkleinert und durch ein Mikrofilter presst.

6. Arzneimittel zur Krebsbekämpfung, dadurch gekennzeichnet, dass es fleischfressenden Pflanzen, bzw. deren Blätter enthält, die zerkleinert und gepresst sind und dass das Exprimat mit NaCl-Lösung zu einer Injektionslösung verdünnt ist.

7. Arzneimittel nach Anspruch 6, dadurch gekennzeichnet, dass das Exprimat mit einer 0,9%igen NaCl-Lösung im ungefähren Verhältnis 10:4 verdünnt ist.

**Claims**

1. A medicament for combating cancer, characterised in that it comprises the sap of carnivorous plants with the exception of Drosera rotundifolia, Pinguicula vulgaris and Pinguicula alpina, optionally together with pharmaceutically-acceptable carriers and/or excipients.

2. A medicament as claimed in Claim 1, characterised in that it comprises the sap of the leaves of the carnivorous plants.

3. A medicament as claimed in Claim 1 or 2, characterised in that it comprises the sap of Dionaea muscipula, also known as Venus' fly-trap.

4. A medicament as claimed in one of Claims 1 to 3, characterised in that it comprises the proteolytic enzymes contained in the carnivorous plants.

5. A method of producing a medicament as claimed in one of Claims 1 to 4, characterised in that the carnivorous plants or their leaves are comminuted and pressed through a microfilter.

6. A medicament for combating cancer, characterised in that it comprises carnivorous plants or their leaves which have been comminuted and pressed, and in that the expressed liquid is diluted with NaCl solution to form an injection solution.

7. A medicament as claimed in Claim 6, characterised in that the expressed liquid is diluted with a 0,9% NaCl solution approximately in the ratio of 10:4.

**Revendications**

1. Médicament contre le cancer, caractérisé en ce qu'il contient le jus de plantes carnivores, à l'exception de Drosera rotundifolia, Pinguicula vulgaris et Pinguicula alpina, le cas échéant avec des excipients et/ou charges pharmaceutiquement acceptables.

2. Médicament suivant la revendication 1, caractérisé en ce qu'il contient le jus des feuilles des plantes carnivores.

3. Médicament suivant l'une quelconque des revendications 1 ou 2, caractérisé en ce qu'il contient le jus de Dionaea muscipula, également nommée gobemouches.

4. Médicament suivant l'une quelconque des revendications 1 à 3, caractérisé en ce qu'il contient les ferments protéolytiques contenus dans les plantes carnivores.

5. Procédé de préparation d'un médicament suivant l'une quelconque des revendications 1 à 4, caractérisé en ce qu'on broie les plantes carnivores, ou leurs feuilles, et en ce qu'on les presse à travers un micro-filtre.

6. Médicament contre le cancer, caractérisé en ce qu'il contient des plantes carnivores ou leurs feuilles, qui sont broyées et pressées, et en ce que l'exprimat est dilué par une solution de NaCl pour donner une solution injectable.

7. Médicament suivant la revendication 6, caractérisé en ce que l'exprimat est dilué avec une solution de NaCl de 0,9% dans le rapport approximatif de 10:4.